# EUROPÄISCHE PATENTANMELDUNG

(11) **EP 3 598 965 A1**
(43) Veröffentlichungstag der Anmeldung: **29.01.2020**
(21) Anmeldenummer: 19175835.8
(22) Anmeldetag: 22.05.2019
(51) Int. Cl.: A61K 8/34, A61K 8/37, A61K 8/92, A61K 8/81, A61K 8/02, A61Q 5/00, A61Q 19/00

(54) **MINERALÖL- UND EMULGATORFREIE ZUBEREITUNG**

(30) Priorität: 26.07.2018 DE 102018212458
(71) Anmelder: Beiersdorf AG, 20253 Hamburg (DE)
(72) Erfinder: Wischhöfer, Svea, 21109 Hamburg (DE); Neckermann, Inken, 25767 Offenbüttel (DE); Eckert, Julia, 22393 Hamburg (DE); Herwig, Katharina, 22529 Hamburg (DE); Schulz, Sabine, 22159 Hamburg (DE); Skubsch, Kerstin, 25497 Prisdorf (DE); Breisig, Hans, 22299 Hamburg (DE); Rosenbaum, Sebastian, 22765 Hamburg (DE)

(57) **Zusammenfassung**

Eine kosmetische oder dermatologische Zubereitung umfassend neben Fettalkohol, ein oder mehreren Lipide, Polyacrylsäurepolymere und Wasser, wobei die Lipide natürlichen Ursprungs sind, zeigen eine erstaunliche Hautpflegeleistung.

## Beschreibung

Die Erfindung ist eine auf nasser Haut anwendbare kosmetische oder dermatologische Zubereitung, die auf Mineralöle und Emulgatoren verzichten kann.

Die Zubereitung eignet sich für die Applikation auf nasser Haut ohne vollständig abgespült zu werden und ermöglicht damit während des Duschens das Eincremen.

Im Markt und im Stand der Technik gibt es zahlreiche kosmetische Zubereitungen, die eine solche Anwendung auf feuchter bzw. nasser Haut ermöglichen. Die als In-shower oder In-Dusch® bezeichneten Produkte zeigen jedoch vielfältige Unterschiede, sowohl hinsichtlich der tatsächlich auf der Haut verbleibenden Lipide als auch hinsichtlich sensorischer und haptischer Eindrücke.

Des Weiteren umfassen zahlreiche Zubereitungen des Standes der Technik Lipide nicht-natürlichen Ursprungs, wie Mineralöle.

Mineralöle hinterlassen zwar mitunter einen hautpflegenden Film. Mineralölhaltige oder auf Mineralöl basierende Rohstoffe werden aber teilweise von den Verbrauchern als negativ bewertet.

Wünschenswert ist es kosmetische Formulierungen bereit zu stellen, die auf nasser Haut anwendbar sind und einen spürbaren und pflegenden Rückstand auf der Haut hinterlassen und anstelle von Mineralölen natürliche Öle umfassen.

Auch wäre die verbesserte biologische Abbaubarkeit von natürlichen Ölen ein wesentlicher Vorteil für für das Produkt und den Verbraucher.

Erfindungsgemäß werden als Mineralöle insbesondere die Öle unter den INCI-Bezeichnungen Mineral oil, Paraffin, Paraffinum Liquidum, Petrolatum, Cera Microcristallina, Microcristalline Wax, Ozokerit verstanden.

In der EP 2986270 A2 wird beschrieben, wie eine kosmetische oder dermatologische Zubereitung erhalten werden kann, die auf nasser Haut aufgetragen werden kann und einen Lipidfilm auf der Haut hinterlässt.

Die Zubereitungen der EP 2986270 A2 sind erhältlich durch
a.) Mischen von ein oder mehreren Polyacrylsäurepolymeren in Wasser unter Energieeintrag,
b.) Aufschmelzen von ein oder mehreren Fettalkoholen und
   b1) mindestens einem zusätzlichem Wachs und/oder
   b2) einem Gemisch aus flüssigen und festen Kohlenwasserstoffen,
   wobei zumindest das Kohlenwasserstoffgemisch einen Schmelzbereich von 5°C bis 75°C, bevorzugt bis 55°C (nach DSC), aufweist,
c.) Zusammenführen der Mischungen a.) und b.) unter Rühren, wobei eine vollständige Homogenisierung vermieden wird, und
d.) gegebenenfalls Zugabe von ein oder mehreren Stoffen gewählt aus der Gruppe der Neutralisierungsmittel, Hautbefeuchtungsmittel, Konservierungsmittel, Öle, Verdicker und Parfüme,
e.) wobei der Zubereitung keine Emulgatoren zugesetzt werden.

In den Zubereitungen der EP 2986270 A2 werden jedoch Mineralöle, wie insbesondere Cera Microcristallina und Paraffinum Liquidum, eingesetzt.

Wünschenswert ist es auch eine alternative Zubereitung zur Anwendung auf nasser oder feuchter Haut zur Verfügung zu stellen.

In der DE 102012221227 A1 werden Hydrodispersionen offenbart. Der Verdickeranteil ist darin jedoch sehr hoch, mit entsprechenden sensorischen Nachteilen.

In der DE 102015207624 A1 werden Hautpflegezubereitungen beschrieben, die eine Öl- und eine Wasserphase umfassen. In der Ölphase ist Natriumpolyacrylat und in der Wasserphase sind Gelbildner enthalten. Die Zubereitungen umfassen keine Emulgatoren.

Im Stand der Technik werden häufig Öle natürlichen Ursprungs neben Ölen nicht-natürlichen Ursprungs, wie Mineralöle, nebeneinander aufgeführt. Jedoch unterscheiden sich die Öle hinsichtlich der Nachhaltigkeitsbetrachtung, der Bioabbaubarkeit sowie in den sensorischen wie haptischen Eigenschaften. Ein Austausch der Öle untereinander mag daher gedankentheoretisch offenbart sein, jedoch würde ein Fachmann aufgrund der Unterschiede diesen Austausch nicht automatisch vollziehen.

Die Erfindung ist eine kosmetische oder dermatologische Zubereitung umfassend
a. ein oder mehrere Fettalkohole,
b. ein oder mehrere Lipide,
c. ein oder mehrere Polyacrylsäurepolymere und
d. Wasser.

Wesentlich ist dabei, dass der Anteil an Mineralölen gewählt aus der Gruppe Mineral oil, Paraffin, Paraffinum liquidum, Petrolatum, Cera Microcristallina, Microcristalline Wax und Ozokerit weniger als 0,1 Gew.% beträgt.

Erfindungsgemäß wird auf den Zusatz von Mineralölen verzichtet. Wenn jedoch Herstell- oder Rohstoffbedingt Mineralöle in die Zubereitungen eingeschleppt werden, so ist der Anteil geringer als 0,1 Gew.%, so dass erfindunsgemäß die Zubereitung dann noch als Mineralölfrei zu gelten. Bevorzugt beträgt der Anteil an Mineralölen in der Zubereitung weniger als 0,01 Gew.%, insbesondere 0 Gew.%, bezogen auf die Gesamtmasse der Zubereitung.

Der Anteil an Emulgatoren beträgt ebenfalls weniger als 0,1 Gew.%, insbesondere weniger als 0,01 Gew.%, bevorzugt 0 Gew.%, bezogen auf die Gesamtmasse der Zubereitung. Auch hier gilt die erfindungsgemäße Zubereitung als emulgatorfrei, wenn durch Herstellprozesse oder Verunreinigungen Emulgatoren bis zu einem Anteil von 0,1 Gew.% eingeschleppt werden.

Die erfindungsgemäß enthaltenen Polyacrylsäurepolymere können emulgierende Eigenschaften aufweisen. Die Polyacrylsäurepolymere sind daher erfindungsgemäß von der Mengenbegrenzung der Emulgatoren ausgenommen, so dass neben den Polyacrylsäurepolymeren weitere Emulgatoren zu maximal 0,1 Gew.%, bzw. weniger als 0,01 Gew.% insbesondere zu 0 Gew.%, enthalten sein können, jeweils bezogen auf die Gesamtmasse der Zubereitung.

Auch auf sog. Feststoffemulgatoren, wie sie beispielsweise in der EP 1992323 A1 beschrieben werden, kann erfindungsgemäß verzichtet werden.

Anders ausgedrückt, neben den Polyacrylsäurepolymeren werden der Zubereitung bevorzugt keine weiteren Emulgatoren zugesetzt.

Natriumpolyacrylat zählt nicht zu den erfindungsgemäßen Polyacrylsäurepolymeren. D.h. die erfindungsgemäßen Zubereitungen enthalten kein Natriumpolyacrylat.

Emulgatoren und Tenside können die Barriereschicht der Haut schädigen. Den Zubereitungen werden daher vorteilhaft weder Emulgatoren noch Tenside zugesetzt.

Weiterer wesentlicher Unterschied zum Stand der Technik ist die Möglichkeit den Wasseranteil in der Zubereitung über 60 Gew.%, insbesondere über 65 Gew.% zu wählen, bezogen auf die Gesamtmasse der Zubereitung.

Ein Vorteil der größeren Wassermenge ist, dass der Herstellprozess, insbesondere im kontinuierlichen Verfahren, energetisch günstiger ist. Sofern mehr Wasser in der Zubereitung enthalten sein kann umso mehr Kaltwasser kann auch zu dosiert werden, was in kontinuierlichen Herstellprozessen zu einer erheblichen Energieeinsparung führen kann. Des Weiteren führt ein Mehr an Wasser zu einer nachhaltigeren Zubereitung, die Rohstoffschonender entwickelt und produziert werden kann.

Die enthaltenen Fettalkohole a. und Lipide b. sind voneinander zu unterscheiden, so dass neben mindestens einem Fettalkohol a. mindestens ein Lipid b. enthalten sein muss, das sich vom Fettalkohol a. unterscheidet.

Die erfindungsgemäße Zubereitung zeichnet sich dadurch aus, dass keine Mineralöle, insbesondere keine Lipide nicht-natürlichen Ursprungs enthalten sind.

Als Lipide werden daher bevorzugt Lipide natürlichen Ursprungs, wie pflanzlichen oder tierischen Ursprungs, eingesetzt. Vorteilhaft werden Lipide eingesetzt, die aus Extraktionen aus Pflanzenbestandteilen gewonnen werden.

Als Lipide werden den erfindungsgemäßen Zubereitungen vorteilhaft ein oder mehrere Lipide zugesetzt, die einen Schmelzbereich von 5 bis 75°C (nach DSC) aufweisen.

DSC (Differential Scanning Calorimetry) ist ein thermisches Verfahren zur Messung von abgegebener/aufgenommener Wärmemenge einer Probe bei isothermer Arbeitsweise, Aufheizung oder Abkühlung (siehe DIN 53765, DIN 51007, ASTM E 474, ASTM D 3418). DSC ist eine vergleichende Messmethode, die die Bestimmung von Wärmemengen physikalischer und chemischer Prozesse ermöglicht. Wenn ein Material seinen physikalischen Zustand ändert, wie z.B. Schmelzen oder Umwandlung einer Kristallform in eine andere oder wenn es chemisch reagiert, wird Wärme dabei aufgenommen oder abgegeben. Diese Wärmemengen sind mit Hilfe der DSC quantitativ messbar. Die Methode verläuft zyklisch, so dass nach der ersten Aufheizkurve ein definiertes Abkühlen stattfindet und anschließend die Probe noch einmal im angegebenen Temperaturbereich aufgeheizt wird. Man erhält somit zweierlei Informationen: In der ersten Aufheizkurve sind alle thermische Effekte inklusiv Vorgeschichte erkennbar. In der zweiten Aufheizkurve ist die Vorgeschichte eliminiert worden und das reine thermische Verhalten der Probe unter definierten Abkühlbedingungen ist auswertbar. Der Schmelzbereich der Lipide zwischen 4,5°C und 75°C nach DSC ist der in der ersten Aufheizkurve ermittelte Bereich.

Bevorzugt werden als Lipide b. ein oder mehrere Lipide aus der Gruppe Cetyl Palmitate, Isopropyl Palmitate, Butyrospermum Parkii Butter, Hydrogenated Vegetable Oil, Rapseedoil, Shea Butter und/oder Coconut oil gewählt.

Bevorzugt wird eine Kombination aus zwei oder mehreren Lipiden gewählt aus der Gruppe Butyrospermum Parkii Butter, Hydrogenated Cocoglyceride und Hydrogenated rapeseed oil gewählt.

Die Gewichtsanteile der bevorzugten Lipide können im Bereich von 50 bis 68 Gew.%, bevorzugt 55 - 65 Gew.% an Butyrospermum Parkii Butter, von 15 bis 40 Gew.%, bevorzugt 20 - 30 Gew.% an Hydrogenated Cocoglyceride und von 2 bis 15 Gew.%, bevorzugt 5 - 10 Gew.% an Hydrogenated rapeseed oil gewählt werden, bezogen auf die Gesamtmasse an Lipiden.

Es ist bekannt, dass unpolare mikrokristalline Lipide in Zubereitungen des Standes der Technik nach dem Abspülen einen hydrophoben Film auf der Haut hinterlassen.

Die erfindungsgemäß bevorzugten polaren, natürlichen Lipide sind hydrophiler als diese unpolaren Lipide. Der Fachmann würde daher beim Austausch der unpolaren Mineralöle durch polarere Lipide, wie erfindungsgemäß bevorzugte, erwarten, dass diese polaren Lipide einfacher mit Wasser von der Haut entfernt werden können und daher kein gewünschter Lipidfilm auf der Haut verbleibt.

Erstaunlicherweise führte aber die erfindungsgemäße Kombination zu einem anderen Ergebnis, indem durchaus ein Hautpflegefilm erzeugt werden konnte, der zudem auch auf der Haut verbleibt ohne sofort mit Wasser abgespült zu werden.

Dies ist ein weiterer Vorteil gegenüber In-shower Zubereitungen des Standes der Technik. Bei diesen Zubereitungen wird ein Abspülen nach dem Applizieren durchweg empfohlen. Dies auch weil ansonsten ein sensorisch inakzeptables Rückstandsgefühl auf der Haut verbleibt.

Erfindungsgemäße Zubereitungen zeigen aber wider Erwarten auch ohne nachträgliches Abspülen ein sensorisch angenehmes Hautgefühl.

Mit der erfindungsgemäßen speziellen Kombinationen aus natürlichen Lipiden konnte ein sensorisch angenehmer Hautfilm in überraschender Weise erzielt werden, wie im Sensorikpanel und mittels Kontaktwinkelmessung gezeigt werden konnte.

Als Fettalkohole werden bevorzugt C14 bis C22 Fettalkohole verwendet. Bevorzugt werden die Fettalkohole gewählt aus der Gruppe linearen Fettalkohole, insbesondere Myristylalkohol (C₁₄H₃₀O), Cetylalkohol (oder Palmitylalkohol) (C₁₆H₃₄O), Stearylalkohol (oder Octadecylalkohol) (C₁₈H₃₈O) sowie Cetylstearylalkohol (Cetearylalkohol),Behenylalkohol, Lanolin Alcohol, ein Gemisch der Alkohole Cetylalkohol (Hexadecanol) und Stearylalkohol (Octadecanol).

Der Anteil an C14-22 Fettalkoholen insgesamt beträgt vorteilhaft 3 bis 14 Gew.%, insbesondere 7 bis 9 Gew.%, bezogen auf die Gesamtmasse der Zubereitung.

Als Fettalkohole a. werden bevorzugt ein oder mehrere Fettalkohole gewählt aus der Gruppe umfassend Myristylalkohol, Stearylalkohol und Cetearylalkohol.

Als Polyacrylsäurepolymere werden die in der Kosmetik bekannten Polymere der Acryl- und/oder Methacrylsäure sowie Acrylat-Crosspolymere verstanden.

Erfindungsgemäß sind Natriumpolyacrylate keine Polyacrylsäurepolymere. Der Anteil an Natriumpolyacrylaten in der Zubereitung beträgt daher bevorzugt weniger als 0,1 Gew.%, insbesondere 0 Gew.%, bezogen auf die Gesamtmasse der Zubereitung.

Besonders bevorzugte Polyacrylsäurepolymere sind neben den Carbomeren diejenigen Acrylat-Crosspolymere, die eine polymere Emulgatorwirkung ausüben.

Die emulgierend wirkenden Polymere sind hauptsächlich Polyacrylsäurepolymere mit hohem Molekulargewicht. Diese emulgierend wirkenden Polyacrylsäurepolymere haben einen kleinen lipophilen Anteil zusätzlich zum hydrophilen Hauptteil. Ganz besonders bevorzugt im Sinne der vorliegenden Erfindung sind Acrylat-Crosspolymere, welche die INCI Bezeichnung "Acrylates/C10-30 Alkyl Acrylate Crosspolymer" haben und unter den Handelsbezeichnungen PemulenTR-1 und Pemulen TR-2 sowie Carbopol 1342, Carbopol 1382 und Carbopol ETD 2020 von der Firma Lubrizol/GF Goodrich erhältlich sind.

Besonders bevorzugt werden die Polyacrylsäurepolymere gewählt aus der Gruppe der Acrylates/C10-30 Alkyl Acrylate Crosspolymere und/oder Carbomere. Insbesondere bevorzugt sind Acrylates/C10-30 Alkyl Acrylate Crosspolyme, Pemulen® TR-1 oder TR-2, z.B. von Lubrizol und Carbopol® 3128 von Lubrizol.

Erfindungsgemäß ist hierbei bevorzugt eine spezifische Kombination aus Polyacrylsäurepolymeren mit emulgierender Wirkung, wie das Pemulen® TR-2 mit anderen Polyacrylsäurepolymeren, wie Carbopol 1382, die die sensorischen Eigenschaften verbessern und die Stabilität der Zubereitung, insbesondere bei höheren Temperaturen, und eine Verbindung mit freiem Wasser gewährleisten.

Besonders bevorzugt ist hierbei eine Kombination aus drei Polyacrylsäurepolymeren, wobei ein Polyacrylsäurepolymer eine emulgierende Wirkung aufweist, wie z.B. das Pemulen TR-1 oder Pemulen TR-2, und die anderen Polyacrylsäurepolymeren, die sensorischen Eigenschaften verbessern und die Stabilität der Zubereitung, insbesondere bei höheren Temperaturen, gewährleisten. So z.B. das Carbopol 1382 und einem Polyacrylsäurepolymer, das die sensorischen Eigenschaften bei Aufnahme von freiem Wasser verbessert (z.B. Carbopol 981).

Vorteilhaft wird bei der Herstellung der erfindungsgemäßen Zubereitung daher bevorzugt mindestens drei Polyacrylsäurepolymere, insbesondere drei Polyacrylsäurepolymere, die sich in ihren Eigenschaften unterscheiden, zugesetzt.

Als Polyacrylsäurepolymere werden insbesondere ein oder mehrere Polymere aus der Gruppe der Acrylates/C10-30 Alkyl Acrylate Crosspolymere und/oder Carbomere gewählt.

Der Anteil an Polyacrylsäurepolymeren insgesamt beträgt vorzugsweise 0,05 bis 2 Gew.%, bevorzugt von 0,2 bis 2 Gew.%, insbesondere 0,3 bis 1 Gew.%, bezogen auf die Gesamtmasse der Zubereitung.

Weiterer Vorteil ist es, wenn die Zubereitung so eingestellt wird, dass die Zubereitung eine dynamische Viskosität im Bereich von 500 bis 10.000 mPas aufweist, insbesondere im Bereich von 2.500 bis 7.000mPas (gemessen mit Rheomat 123 der fa. proRheo bei 25°C Spindel 1).

Das erfindungsgemäße Gemisch aus Fettalkoholen (a.), Lipiden (b.) und Polyacrylsäurepolymeren (c.) erzeugt vorteilhaft in der Zubereitung Partikel. Diese Partikel weisen eine Partikelgröße von maximal 1000 µm, insbesondere im Bereich von 1 - 500 µm auf.

Die Partikelbildung wird im Herstellprozess, durch die Mischung als Schmelze HOT/HOT oder HOT/COLD und durch Scherkräfte vor dem Neutralisieren verwirklicht.

Die Definition des Begriffs "Partikelgröße" ist wie folgt zu verstehen. Partikel sind dreidimensionale Gebilde, die fest und/oder flüssig sein können und eine von der Umgebung abgrenzbare Struktur aufweisen.

Die Partikel bestehen im Wesentlichen aus der Fettphase. Sie unterscheidet sich von der Umgebung, da diese Partikel durch das Gelnetzwerk (Umgebung) stabilisiert werden. Dabei kann ein Teil der Fettphase sich auch in dem Gelnetzwerk befinden. Entscheidend ist daher nicht eine 100%igen Trennung zwischen Gelnetzwerk (Umgebung) und Fettphase sondern die erfindungsgemäße Bildung der körnigen Struktur mit einer Partikeldichte von ein oder mehreren Kristalliten mit einer Partikelgröße insbesondere von 1 bis 500 µm auf 4 mm² Fläche der Fettphase der Zubereitung.

Drei Parameter (Länge, Breite, Höhe) sind erforderlich, um eine vollständige Beschreibung von Partikelgrößen geben zu können. Es ist daher eigentlich schwer möglich, ein Partikel durch Angabe einer einzigen Zahl zu beschreiben, die der Partikelgröße entspricht. Bei der Mehrzahl der Methoden zur Größenbestimmung wird deshalb davon ausgegangen, dass das zu messende Material kugelförmig ist, da eine Kugel die einzige Form ist, die mittels einer einzigen Zahl (dem Durchmesser) beschrieben werden kann.

Umfasst das Produkt kugelförmige Partikel, so ist die Partikelgröße durch die Angabe des Kugeldurchmessers eindeutig definiert, wie erfindungsgemäß bevorzugt im Bereich von 5 bis 500 µm. Die Mehrzahl der feinkörnigen Strukturen bzw. Kristallite besteht aber nicht aus Kugeln, sondern aus mehr oder weniger unregelmäßig geformten Partikeln, die in einem Extremfall nadelförmig, im anderen plättchenförmig sein können. Die Dispersitätseigenschaft der Einzelpartikeln lässt sich hier durch das Partikelvolumen und zusätzliche Parameter wie Sphärizität (Kugelähnlichkeit) beschreiben. Anstelle der für Kugeln einparametrigen Funktion erhält man eine vielparametrige Dispersitätsfunktion, deren Bestimmung mit großem messtechnischem Aufwand verbunden ist. Ein derartiger Aufwand ist nur dann gerechtfertigt, wenn er dazu dient, wesentliche Informationen über Produkteigenschaften zu gewinnen.

Daher beschränkt man sich in der Praxis normalerweise und auch erfindungsgemäß auf die Angabe einer einparametrigen Verteilungsfunktion für die Partikelgröße, dem Durchmesser für angenommene Kugelform der Partikel.

Zur Charakterisierung einer Partikelgrößenverteilung werden die Werte D₁₀, D₅₀ und D₉₀ verwendet. Der Wert von D₅₀ ist als mittlere Partikelgröße definiert gemäß DIN 13320.

Die als körnige Struktur, Partikel, Kristallite oder Griesigkeit bezeichnete Textur der erfindungsgemäßen Zubereitung umfasst daher im Wesentlichen Fettphasenbestandteile, die sich optisch von der Umgebung unterscheiden.

Bei mikroskopischem Vergleich der erfindungsgemäßen Formel ergab sich, dass sich kleinere kristalline Partikel bildeten, als die im Stand der Technik bekannten Zubereitungen mit Cera Microcristallina. Durch die vielen kleinen Kristalle wird die Haut genauso gut benetzt, wie mit den großen Kristallen. Dies ist umso erstaunlicher, da dennoch ein sensorisch angenehmer Rückstand nach dem Duschen auf der Haut verbleibt.

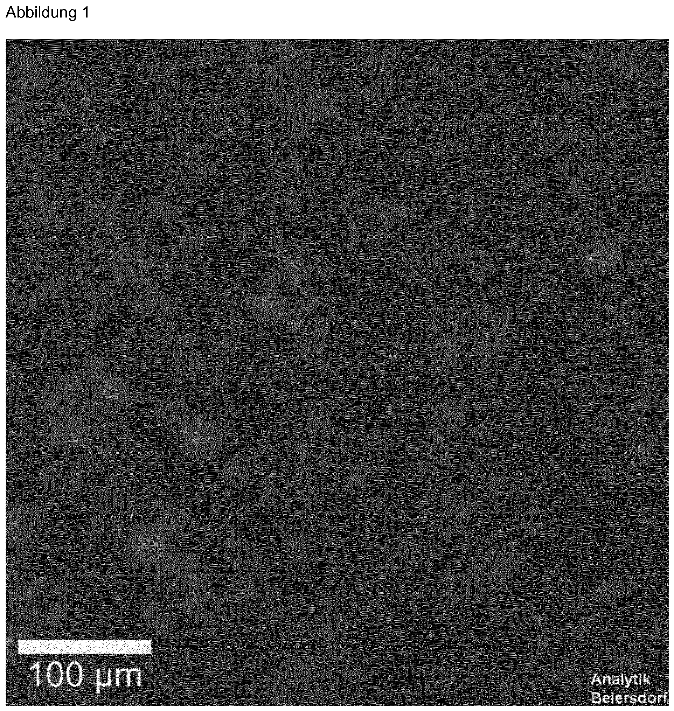

Abbildung 1 zeigt eine mikroskopische Aufnahme der Zubereitung mit der Kombination aus Butyrospermum Parkii Butter, Hydrogenated Coco-Glycerides, Hydrogenated Rapseedoil und gibt einen Hinweis auf die Struktur/Kristallform. Die Kristallgröße ist lt. mikroskopischem Bild kleiner als die Tröpfchengröße (D50 = 10,8 µm).

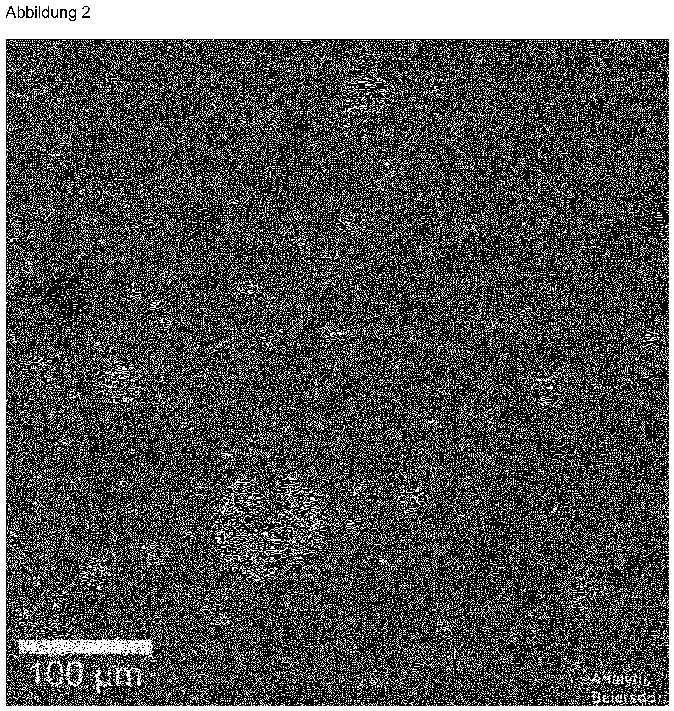

Abbildung 2 zeigt die mikroskopische Aufnahme mit Cera Microcristallina. Die Kristallgrößenverteilung ist breiter und inhomogener. Die Kristallgröße ist lt. mikroskopischem Bild kleiner als die Tröpfchengröße (D50 = 13,7 µm) oder entspricht dieser.

Die erfindungsgemäßen Zubereitungen hinterlassen aufgetragen auf der nassen Haut überraschenderweise einen pflegenden Rückstand nach dem Abspülen.

Dies war umso mehr erstaunlich, da die erfindungsgemäße Zubereitung gegenüber den Zubereitungen des Standes der Technik mehr Wasser bzw. weniger Lipide umfasst.

Wie die Mikroskopiebilder zeigen, ist die erfindungsgemäße Zubereitung glatt bis grießig und zeigt eine gleichmäßige Tröpfchenverteilung, wohingegen die Zubereitungen mit CeraMicrocristallina große Vaseline-Partikel zeigt.

Trotz der geringeren Fettphase werden die gleichen Pflege Eigenschaften erzielt und die Partikel, die Oilbeads, tragen wesentlich zur Verbesserung des Hautbildes bei.

Die Verwendung der Zubereitung auf feuchter oder nasser Haut ist daher prädestiniert.

Ebenso können die erfindungsgemäßen Zubereitungen als Haarbehandlungsmittel verwendet werden. Überraschend wirken erfindungsgemäße Zubereitungen in der Haarbehandlung pflegend auf die Haare. Eine Haarkur, ggf. mit entsprechenden Haarpflegewirkstoffen, ist daher eine weitere bevorzugte Anwendungsform.

Vorteilhaft ist auch hier eine Basis von natürlichen Rohstoffen, die einen pflegenden und/der schützenden Film auf den Haaren hinterlassen.

Die erfindungsgemäßen Zubereitungen sind weiterhin bevorzugt auch frei von Tensiden.

Den erfindungsgemäßen Zubereitungen können ein oder mehrere Hautbefeuchtungsmittel zugesetzt werden. Vorteilhaft werden den Zubereitungen 1 Gew.% bis zu 30 Gew.%, insbesondere 1 Gew.% bis 20 Gew.%, insbesondere 3 bis 6 Gew.%, bezogen auf die Gesamtmasse der Zubereitung, eines oder mehrerer Hautbefeuchtungsmittel zugesetzt. Vorteilhaft wird Glycerin als Befeuchtungsmittel gewählt.

Einen hohen Anteil an Glycerin im Bereich bis zu 30 Gew.% zu wählen hat den Vorteil, dass hohe Mengen Glycerin einen Wärmeeffekt erzeugen, der für den Anwender angenehm sein kann.

Es könnte erwartet werden, dass aufgrund des Zusatzes an Hautbefeuchtungsmitteln (moisturizern), die Sensorik, Haptik und vor allem die Pflegeeigenschaften der erfindungsgemäßen Zubereitungen nochmals verbessert werden.

Erstaunlicherweise sind die Hautbefeuchtungsleistungen der erfindungsgemäßen Zubereitungen aber auch ohne zusätzliche Hautbefeuchtungsmittel überraschend gut. Dies lässt sich mit dem sich bildenden okklusiven Film und der relativ kleinen Ölpartikel erklären.

Es ist daher ebenso bevorzugt, dass den Zubereitungen kein zusätzliches Hautbefeuchtungsmittel zugesetzt wird um dennoch eine ausreichende Hautbefeuchtungsleitung zu erzielen.

Die kosmetischen oder dermatologischen Zubereitungen gemäß der Erfindung können ferner kosmetische Hilfsstoffe und Wirkstoffe enthalten, wie sie üblicherweise in solchen Zubereitungen verwendet werden, z. B. Wirkstoffe, Konservierungsmittel, Konservierungshelfer, Bakterizide, Farbstoffe und Farbpigmente, anfeuchtende und/oder feuchthaltende Substanzen, Verdicker oder andere übliche Bestandteile einer kosmetischen oder dermatologischen Formulierung wie Polyole, Polymere, Schaumstabilisatoren, organische Lösungsmittel oder Silikonderivate, sofern sie nicht erfindungsgemäß ausgeschlossen sind.

Als zusätzliche Rohstoffe können vorteilhaft den erfindungsgemäßen Zubereitungen zugesetzt werden
- UV-Filter
- Ingwer Extract
- Guarana Extract
- Grape Seed oil
- Vitamine, insbesondere Vitamin E, B5, Vitamin C
- Antioxidantien, insbesondere Tocopherolacetat, BHT

Die erfindungsgemäßen Zubereitungen sind bevorzugt frei von Parfüm und/oder Polyethylenglykolen (PEG's).

Der Anteil an Verdickern wird bevorzugt auf bis zu 0,5 Gew.%, bevorzugt 0,4 Gew.%, bezogen auf die Gesamtmasse der Zubereitung, begrenzt.

Im Herstellungsprozess und der Phasenvorbereitung hat sich erfindungsgemäß ein weiterer Vorteil ergeben, so dass sich ein Einfluss auf die Stuktur der produzierten Zubereitungen ergibt.

Besonders geeignet für den Herstellprozess zeigte sich, wenn die Polyacrylsäurepolymere in der flüssigen Lipidphase, z.B. Sheabutter, angeschlämmt werden und nicht, wie im Stand der Technik, zunächst in der Wasserphase eingearbeitet werden.

Das Aufschlämmen ist dabei der erste Schritt des Verfahrens, wobei ein Gemisch in einer geeigneten Flüssigkeit aufgewirbelt, gelockert, dispergiert wird.

Die Vorteile sind, dass sich keine Agglomerate bilden. Die Klumpenbildung der verschiedenen Polymere bei der Einarbeitung ins Wasser wird verringert bzw. ganz vermieden. Damit ist eine bessere Verteilung in der gesamten Wasserphase gegeben und die Scherkräfte bei der Solubilisation werden vermindert.

Die Polyacrylsäurepolymere werden in einem Teil oder der gesamtem aufgeschmolzenen Fettphase, Komponenten a. Fettalkohole und b. Lipide, aufgeschlämmt und anschließend mit Wasser vermischt und mit ggf. Hautbefeuchtungsmittel (Glycerin) unter Energieeintrag (Rühren) gemischt, wobei eine vollständige Homogenisierung vermieden wird. Anschließend erfolgt eine Neutralisierung der Polyacrylsäuren bei 75-25°C, bevorzugt 45-30°C, bevorzugt mit Natronlauge.

Die erfindungsgemäße kosmetische oder dermatologische Zubereitung ist dabei auch durch diese Herstellparameter charakterisiert um die gewünschten Eigenschaften der kleineren Öl-Beads zu erreichen und leztendlich damit auch die gewünschte Produktperformance zu erzielen. Die erfindungsgemäße kosmetische oder dermatologische Zubereitung ist daher erhältlich nach einem Verfahren gekennzeichnet durch
- Aufschlämmen von ein oder mehreren Polyacrylsäurepolymeren (c.) in einem Teil einer Fettphase oder der gesamten aufgeschmolzenen Fettphase, gebildet aus ein oder mehreren Fettalkoholen (a.) und ein oder mehreren Lipiden (b.),
- Vermischen mit Wasser,
- Mischen unter Energieeintrag (Rühren) und ggf. Zugabe von Hautbefeuchtungsmitteln, wobei eine vollständige Homogenisierung vermieden wird, und
- anschließender Neutralisierung der Polyacrylsäuren bei 75 bis 25°C,
   wobei
- der Anteil an Mineralölen gewählt aus der Gruppe Mineral oil, Paraffin, Paraffinum liquidum, Petrolatum, Cera Microcristallina, Microcristalline Wax und Ozokerit weniger als 0,1 Gew.%,
- der Anteil an Emulgatoren, ausser den Polyacrylsäurepolymeren, weniger als 0,1 Gew.% und
- der Anteil an Wasser mehr als 60 Gew.% beträgt, jeweils bezogen auf die Gesamtmasse der Zubereitung, gewählt wird.

Überraschenderweise hinterlässt die Mineralöl-freie Formel ein gepflegtes weiches Hautgefühl. Nach dem Einziehen bleibt ein Pflegefilm auf der Haut.

In einem Paneltest wurden verschiedene Eigenschaften der erfindungsgemäßen Zubereitung gegenüber einer Stand der Technik In-shower Formulierungen überprüft.

Geschulte Paneltester gaben auf einer Skala von 0 bis 10 entsprechende Beurteilungen ab.

| Procedere | Eigenschaft (0 - 10) | In-shower mit Mineralöl und CeraMicrocristallina (Nivea In-shower) | Beispiel 1 |
|---|---|---|---|
| Auf die Handfläche auftragen und beurteilen | Fließeigenschaften (langsam-schnell) | 2 | 2 |
| Auftrag auf nassem Unterarm | Verteilbarkeit (schwer - einfach) | 7 | 7 |
| | Spreitbarkeit, Reichhaltigkeit (wässrig - reichhaltig) | 6 | 7 |
| Abspülen, nasse Haut | Sichtbare Rückstände (nein - ja) | 0 | 0 |
| | Gleitfähigkeit (stumpf - gleitend) | 5 | 7 |
| | Taktile Rückstände (keine - viel) | 3 | 5 |
| Getrocknete Haut | Klebrigkeit (nein - ja) | 0 | 0 |
| | Gleitfähigkeit (stumpf - gleitend) | 5 | 6 |
| | Weichheit (rau - weich) | 5 | 6 |
| | Taktile Rückstände (keine - viel) | 2 | 4 |

Es zeigt sich, dass erfindungsgemäße Zubereitungen hinsichtlich der Pflegeeigenschaften und haptischen wie sensorischen Eindrücken genauso gut und vielfach besser abschneiden als Zubereitungen des Standes der Technik. Insbesondere vermitteln die erfindungsgemäßen Zubereitungen einen verbesserten Eindruck, nach dem Trocknen sich als eingecremt zu fühlen, indem ein spürbarer Pflegefilm verbleibt.

Erstaunlich sind diese Eigenschaften auch deswegen, da erfindungsgemäße Zubereitungen mehr Wasser umfassen als Zubereitungen des Standes der Technik und daher eigentlich eine verschlechterte Rückstandsbildung zu erwarten gewesen wäre.

Der sich wider erwartende gebildete okklusive Pflegefilm verbleibt auf der Haut und wird nicht vollständig abgespült.

Weitere optionale Eigenschaften der erfindungsgemäßen Zubereitungen sind bzw. ergeben sich durch Zusatz an Elektrolyten.

Der Zusatz an Elektrolyten hat eine weitere Stabilisierung der Zubereitung zur Folge. Die zugesetzten Elektrolyten tragen dazu bei, dass die Hautbefeuchtung signifikant erhöht wird. Durch den Zusatz insbesondere ein und zweiwertiger Salzen wird zudem die Viskosität stabilisiert.

Die erfindungsgemäßen Zubereitungen lassen sich vorteilhaft als Spray Applikation, Pumpspray oder Aerosol applizieren.

Die erfindungsgemäßen Zubereitungen lassen sich sowohl auf feuchter oder nasser Haut als auch auf trockner Haut anwenden und führen zu den aufgeführten Hautpflegewirkungen. Ein Abspülen mit Wasser nach der Applikation ist möglich und bevorzugt, jedoch nicht zwingend erforderlich, so dass auch hier die erfindungsgemäße Zubereitung breitere Anwendungsmöglichkeiten für den Konsumenten bietet.

Überraschenderweise hinterlässt die erfindungsgemäße Mineralöl-freie Zubereitung ein gepflegtes, weiches Hautgefühl auch wenn die Haut nach der Applikation nicht abgespült wird. Ein zu erwartendes klebriges Hautgefühl, wie es die Stand-der-Technik Zubereitungen nach der Applikation und ohne Abspülen zeigen, stellt sich erfindungsgemäß nicht ein.

Eine Verwendung der erfindungsgemäßen Zubereitung zur Haut- als auch Haarpflege ist prädestiniert.

Entsprechend können erfindungsgemäße Haut- als auch Haarbehandlungsmittel gestaltet sein, wie beispielhaft nachstehend ausgeführt.

Die Zahlenangaben sind auf Gewichtsanteile jeweils bezogen auf die Gesamtmasse der Zubereitung.

### Beispiel 1

| INCI | m [%] | |
|---|---|---|
| Aqua | 67,45 | Wasserphase |
| Glycerin + Aqua | 5,10 | |
| Summe [%] | 72,55 | |
| | | Fettphase |
| Myristyl Alcohol | 1,00 | |
| Cetearyl Alcohol | 4,00 | |
| Cetyl Palmitate | 3,00 | |
| Stearyl Alcohol | 2,00 | |
| 55-65% Butyrospermum Parkii Butter 20-30% Hydrogenated Cocoglyceride 5-10% Hydrogenated rapeseed oil 0,001% Citric acid | 15,00 | |
| Summe [%] | 25,00 | |
| Acrylates/C10-30 Alkyl Acrylate Crosspolymer | 0,18 | Polymere |
| Carbomer | 0,03 | |
| Acrylates/C10-30 Alkyl Acrylate Crosspolymer | 0,15 | |
| Summe [%] | 0,36 | |
| Phenoxyethanol | 0,80 | Konservierung |
| Aqua + Sodium Hydroxide | 0,24 | Neutralisierung |
| Prunus Amygdalus Dulcis Oil | 0,35 | Parfüm |
| | | |
| Parfum | 0,70 | |
| Summe [%] | 1,05 | |
| Summen ges: | 100,00 | |

## Patentansprüche

1. Kosmetische oder dermatologische Zubereitung umfassend
a. ein oder mehrere Fettalkohole,
b. ein oder mehrere Lipide, das bzw. die sich von der Gruppe der Fettalkohole (a.) unterscheiden,
c. ein oder mehrere Polyacrylsäurepolymere und
d. Wasser,
**dadurch gekennzeichnet, dass**
der Anteil an Mineralölen gewählt aus der Gruppe Mineral oil, Paraffin, Paraffinum liquidum, Petrolatum, Cera Microcristallina, Microcristalline Wax und Ozokerit weniger als 0,1 Gew.%,
der Anteil an Emulgatoren, ausser den Polyacrylsäurepolymeren, weniger als 0,1 Gew.% und
der Anteil an Wasser mehr als 60 Gew.% beträgt, jeweils bezogen auf die Gesamtmasse der Zubereitung.

2. Zubereitung nach Anspruch 1 **dadurch gekennzeichnet, dass** als Lipide b. ein oder mehrere Lipide pflanzlichen oder tierischen Ursprungs gewählt werden.

3. Zubereitung nach Anspruch 1 oder 2 **dadurch gekennzeichnet, dass** als Lipide b. ein oder mehrere Lipide aus der Gruppe der Lipide mit einem Schmelzbereich von 5 bis 75°C (nach DSC) ausgewählt werden.

4. Zubereitung nach einem der vorstehenden Ansprüche **dadurch gekennzeichnet, dass** als Lipide b. ein oder mehrere Lipide aus der Gruppe Cetyl Palmitate, Isopropyl Palmitate, Butyrospermum Parkii Butter, Hydrogenated Vegetable Oil, Rapseedoil, Shea Butter und/oder Coconut oil ausgewählt werden.

5. Zubereitung nach einem der vorstehenden Ansprüche **dadurch gekennzeichnet, dass** als Fettalkohole a. ein oder mehrere Fettalkohole gewählt werden aus der Gruppe umfassend Myristylalkohol, Stearylalkohol, Cetearylalkohol.

6. Zubereitung nach einem der vorstehenden Ansprüche **dadurch gekennzeichnet, dass** als Polyacrylsäurepolymere c. ein oder mehrere Polymere aus der Gruppe der Acrylates/C10-30 Alkyl Acrylate Crosspolymere und/oder Carbomere gewählt werden.

7. Zubereitung nach einem der vorstehenden Ansprüche **dadurch gekennzeichnet, dass** der Anteil an Polyacrylsäurepolymeren c. im Bereich von 0,05 bis 2 Gew.% gewählt wird, insbesondere im Bereich von 0,3 bis 1 Gew.%, bezogen auf die Gesamtmasse der Zubereitung.

8. Zubereitung nach einem der vorstehenden Ansprüche **dadurch gekennzeichnet, dass** der Anteil an Natriumpolyacrylaten in der Zubereitung weniger als 0,1 Gew.%, insbesondere 0 Gew.%, bezogen auf die Gesamtmasse der Zubereitung, beträgt.

9. Zubereitung nach einem der vorstehenden Ansprüche **dadurch gekennzeichnet, dass** der Anteil an Wasser mehr als 65 Gew.% beträgt, bezogen auf die Gesamtmasse der Zubereitung.

10. Zubereitung nach einem der vorstehenden Ansprüche **dadurch gekennzeichnet, dass** der Anteil an Mineralölen weniger als 0,01 Gew.% beträgt, bezogen auf die Gesamtmasse der Zubereitung.

11. Zubereitung nach einem der vorstehenden Ansprüche **dadurch gekennzeichnet, dass** die Zubereitung eine dynamische Viskosität im Bereich von 500 bis 10.000 mPas aufweist, insbesondere im Bereich von 2.500 bis 7.000mPas (25°C, Rheomat 123 (proRheo), Spindel 1).

12. Zubereitung nach einem der vorstehenden Ansprüche umfassend Partikel mit einer Partikelgröße D50 von maximal 1000 µm, gebildet aus dem Gemisch aus Fettalkoholen (a.), Lipiden (b.) und Polyacrylsäurepolymeren (c.).

13. Zubereitung nach Anspruch 12 **dadurch gekennzeichnet, dass** die Partikelgröße im Bereich D50 von 5 - 500 µm liegt.

14. Verwendung der Zubereitung nach einem der vorstehenden Ansprüche als Hautpflegemittel.

15. Verwendung nach Anspruch 14 auf feuchter oder nasser Haut.

16. Verwendung der Zubereitung nach einem der vorstehenden Ansprüche 1 bis 13 als Haarbehandlungsmittel.

17. Verfahren zur Herstellung einer kosmetischen oder dermatologischen Zubereitung nach einem der Ansprüche 1 bis 13 **gekennzeichnet durch**
- Aufschlämmen von ein oder mehreren Polyacrylsäurepolymeren (c.) in einem Teil der Fettphase oder der gesamten aufgeschmolzenen Fettphase, gebildet aus ein oder mehreren Fettalkoholen (a.) und ein oder mehreren Lipiden (b.),
- Vermischen mit Wasser,
- Mischen unter Energieeintrag (Rühren) und ggf. Zugabe von Hautbefeuchtungsmitteln, wobei eine vollständige Homogenisierung vermieden wird, und
- anschließender Neutralisierung der Polyacrylsäuren bei 75 bis 25°C.

18. Kosmetische oder dermatologische Zubereitung erhältlich nach einem Verfahren **gekennzeichnet durch**
- Aufschlämmen von ein oder mehreren Polyacrylsäurepolymeren (c.) in einem Teil einer Fettphase oder der gesamten aufgeschmolzenen Fettphase, gebildet aus ein oder mehreren Fettalkoholen (a.) und ein oder mehreren Lipiden (b.),
- Vermischen mit Wasser,
- Mischen unter Energieeintrag (Rühren) und ggf. Zugabe von Hautbefeuchtungsmitteln, wobei eine vollständige Homogenisierung vermieden wird, und
- anschließender Neutralisierung der Polyacrylsäuren bei 75 bis 25°C,
wobei
- der Anteil an Mineralölen gewählt aus der Gruppe Mineral oil, Paraffin, Paraffinum liquidum, Petrolatum, Cera Microcristallina, Microcristalline Wax und Ozokerit weniger als 0,1 Gew.%,
- der Anteil an Emulgatoren, ausser den Polyacrylsäurepolymeren, weniger als 0,1 Gew.% und
- der Anteil an Wasser mehr als 60 Gew.% beträgt, jeweils bezogen auf die Gesamtmasse der Zubereitung, gewählt wird.
